(19) Europäisches Patentamt
European Patent Office
Office européen des brevets

(11) Veröffentlichungsnummer: **0 411 567 A1**

(12) # EUROPÄISCHE PATENTANMELDUNG

(21) Anmeldenummer: **90114687.8**

(51) Int. Cl.[5]: **C07D 243/38**, A61K 31/55

(22) Anmeldetag: **31.07.90**

(30) Priorität: **31.07.89 DD 331279**

(43) Veröffentlichungstag der Anmeldung: **06.02.91 Patentblatt 91/06**

(84) Benannte Vertragsstaaten: **AT BE CH DE ES FR GB GR IT LI NL SE**

(71) Anmelder: **ARZNEIMITTELWERK DRESDEN GMBH I.G.**
**Wilhelm-Pieck-Strasse 35**
**O-8122 Radebeul(DE)**

(72) Erfinder: **Rüger, Carla, Dr.**
**Zillerstrasse 7**
**DDR-8122 Radebeul(DD)**
Erfinder: **Sauer, Wolfgang, Dr.**
**Platanenstrasse 39**
**DDR-8023 Dresden(DD)**
Erfinder: **Lohmann, Dieter, Dr.**
**Hoflössnitzstrasse 30**
**DDR-8122 Radebeul(DD)**
Erfinder: **Poppe, Hildegard Dr.**
**Kieler Strasse 6**
**DDR-8080 Dresden(DD)**
Erfinder: **Bartsch, Reni, Dr.**
**Stephensonstrasse 11**
**DDR-8045 Dresden(DD)**
Erfinder: **Lichoserstov, Arkadij Michajlovic, Dr.**
**Kamenki, II, 1**
**156 Moskau(SU)**
Erfinder: **Kaverina, Natalja Veniaminovna, Prof. Dr.**
**Ul. W. Ulbricht 3, Wohnung 32**
**125 252 Moskau(SU)**
Erfinder: **Skoldinov, Aleksandr Petrovic, Prof.Dr.**
**Casovaja Ul. 25, Korpus 2, Wohnung 99**
**125 315 Moskau(SU)**
Erfinder: **Grigoreva, Ekaterina Klementevna, Dr.**
**Ul. Sirokaja 19, Korpus 19, Wohnung 193**
**129 224 Moskau(SU)**
Erfinder: **Talmachova, Ekaterina A., Dr.**
**Ul. Staroslobodska 14, Wohnung 77**
**Moskau(SU)**
Erfinder: **Lyskovzev, Valentin Viktorovic, Dr.**
**Filevski-Boulevard 17, Wohnung 475**
**121 601 Moskau(SU)**
Erfinder: **Stawrowskaja, Alwina W.**
**Ul. Flotskaja 22, Wohnung 125**
**Moskau(SU)**
Erfinder: **Chichkanov, Gennadi G., Dr.**
**Prospekt Vernadsky 123, Wohnung 104**
**Moskau(SU)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **Neue 5-(omega-Aminoacyl)-5,10-dihydro-11H-dibenzo[b,e][1,4]-diazepin-11-one, Verfahren zu ihrer Herstellung und ihre Verwendung als Arzneimittel.**

(57) Die Erfindung betrifft 5-(ω-Aminoacyl)-5,10-dihydro-11H-dibenzo [b,e] [1,4]diazepin-11-one der Formel I

EP 0 411 567 A1

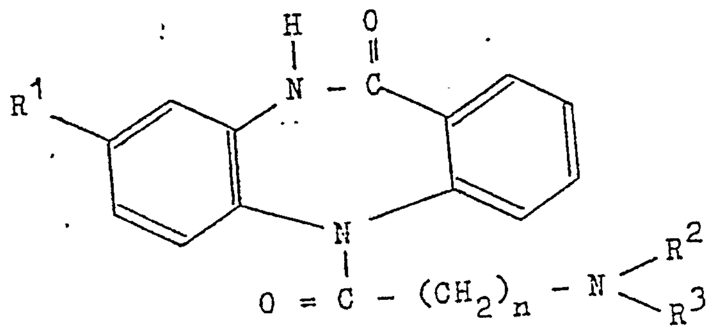

(I),

worin bedeuten:
$R^1$ H oder Cl
$R^2$, $R^3$, H oder $C_{1-3}$ -Alkyl oder zusammen mit dem N-Atom, Morpholino/oder N-Methylpiperazino
und
n 3, 4, 5 oder 6,
wobei, wenn $R^2$ H ist, $R^3$ auch Cyclohexyl bedeuten kann,
und ihre Salze, Verfahren zu ihrer Herstellung sowie ihre pharmazeutische Verwendung.

Die Verbindungen I besitzen antiarrhythmische und anticholinerge Wirksamkeit und geringe Toxizität und eignen sich zur Behandlung von Arrhythmien und insbesondere Bradyarrhythmien.

## NEUE 5 (ω-AMINOACYL)-5,10-DIHYDRO-11H-DIBENZO[B,E][1,4]-DIAZEPIN-11-ONE, VERFAHREN ZU IHRER HERSTELLUNG UND IHRE VERWENDUNG ALS ARZNEIMITTEL

Die Erfindung betrifft neue 5-(ω-Aminoacyl)-5,10-dihydro-11H-dibenzo [b,e] [1,4] diazepin-11-one der allgemeinen Formel I,

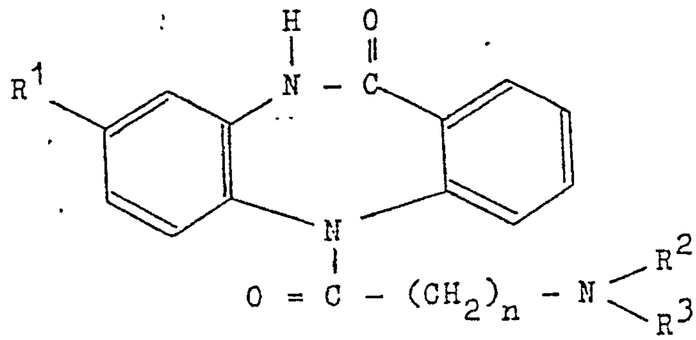

(I),

worin bedeuten:

$R^1$ Wasserstoff oder Chlor,

$R^2$ und $R^3$, die gleich oder verschieden sein können, Wasserstoff, geradkettiges oder verzweigtes $C_{1-3}$-Alkyl oder zusammen mit dem N-Atom, an das sie gebunden sind, einen Morpholino- oder N-Methylpiperazinorest

und

n 3, 4, 5 oder 6,

wobei, wenn $R^2$ Wasserstoff ist, $R^3$ auch Cyclohexyl bedeuten kann,

und

ihre Salze, mit physiologisch verträglichen anorganischen und organischen Säuren sowie Verfahren zu ihrer Herstellung und die Verwendung dieser Verbindungen als oder in Arzneimitteln sowie entsprechende pharmazeutische Mittel.

Die Verbindungen der Formel I eignen sich insbesondere zur Behandlung von Arrhythmien und cholinergen Störungen, besonders in der Humanmedizin.

Es sind bereits 5,10-Dihydro-11H-dibenzo [b,e] [1,4] diazepin-11-one bekannt, die in 5-Stellung eine Aminoacetyl-oder einen Aminopropionylrest aufweisen (DE-A-19 36 670, 20 22 790, 20 65 570, 17 95 176, 19 31 487, 30 28 001, 32 04 157, 32 04 185, BE-A-753 664, DD-A-236 731, EP-A-44 989).

Diese Verbindungen besitzen eine antiulceröse und antisekretorische Wirkung.

Weitere in 5-Stellung mit einem Aminoacetyl- oder Aminopropionylrest substituierte 5,10-Dihydro-11H-dibenzo [b,e]-[1,4]diazepin-11-one wurden von A. M. Monro und Mitarbeitern in J. med. chem. 6 , 255 ohne Angabe pharmakologischer Eigenschaften beschrieben.

Weiterhin sind in 11-Stellung substituierte 5,11-Dihydro-6H-pyrido[2,3-b] [1,4]benzodiazepin-6-one mit Einfluß auf die Herzfrequenz bekannt (DE-A-34 09 237, 35 23 002).

Die meisten Antiarrhythmika der Klasse I wie Lidocain, Ethmozin oder Propafenon haben neben der antiarrhythmischen Wirkung die Eigenschaft, die Herzfrequenz zu senken. Sie sind daher zur Behandlung von Tachyarrhythmien geeignet.

Bei Arrhythmien mit geringer Schlagfolge (Bradyarrhythmien) können diese Mittel deshalb nur in Verbindung mit Atropin zur Anwendung gelangen, damit eine weitere Senkung der Herzfrequenz verhindert wird.

Mittel, die sowohl antiarrhythmisch als auch anticholinerg wirken und damit zur Behandlung von Bradyarrhythmien eingesetzt werden können, sind nur bedingt vorhanden und haben den Nachteil, daß sie entweder überwiegend anticholinerg (Atropin, Ipratropium bromid) oder überwiegend antiarrhythmisch (Chinidin, Disopyramid) wirken.

Es bestand daher ein Bedürfnis, Wirkstoffe zu finden, die ein möglichst ausgewogenes Verhältnis von antiarrhythmischer und anticholinerger Wirkung besitzen und damit zur Therapie von Bradyarrhythmien (Arrhythmien bei langsamer Schlagfolge des Herzens) geeignet sind.

Der Erfindung liegt die Aufgabe zugrunde, neue 5-(ω-Aminoacyl)-5,10-dihydro-11H-dibenzo[b,e] [1,4] diazepin-11-one mit wertvollen pharmakologischen Eigenschaften und Verfahren zu ihrer Herstellung sowie

ihre pharmazeutische Verwendung anzugeben.

Die Aufgabe wird anspruchsgemäß gelöst. Die abhängigen Ansprüche betreffen vorteilhafte Ausführungsformen.

Die Verbindungen der allgemeinen Formel I sind hochwirksame Herz-Kreislauf-Pharmaka, die eine ausgesprochen starke antiarrhythmische Wirkung besitzen und deshalb zur Therapie von Herzrhythmusstörungen eingesetzt werden können.

Während die meisten Antiarrhythmika wie Lidocain und Chinidin die Herzfrequenz senken, halten die Verbindungen der allgemeinen Formel I aufgrund der gleichzeitig vorhandenen anticholinergen Eigenschaft den bestehenden Herzrhythmus über längere Zeit auf gleichem Niveau.

Die Kombination von antiarrhytmischer und anticholinerger Wirkung bei den Verbindungen der allgemeinen Formel I ist für die Herstellung von Arzneimitteln von großem Nutzen, die zur Behandlung von bradykarden Rhythmusstörungen eingesetzt werden können. Die Verbindungen übertreffen in ihrer Wirkungsstärke im Tierexperiment Präparate wie Chinidin und Lidocain.

Besonders vorteilhafte erfindungsgemäße Verbindungen sind

5-(6-Dimethylamino-capronyl )-5,10-dihydro-11H-dibenzo-[b,e] [1,4]diazepin-11-on
5-(4-Diethylamino-butyryl)-5,10-dihydro-11H-dibenzo [b,e][1,4]diazepin-11-on
5-(6-Diethylamino-capronyl)-5,10-dihydro-11H-dibenzo [b,e] [1,4]diazepin-11-on
8-Chlor-5-(4-diethylamino-butyryl)-5,10-dihydro-11H-dibenzo[b,e] [1,4]-diazepin-11-on
8-Chlor-5-(5-diethylamino-valeryl)-5,10-dihydro-11H-dibenzo [b,e] [1,4]diazepin-11-on
8-Chlor-5-(6-diethylamino-capronyl)-5,10-dihydro-11H-dibenzo [b,e] [1,4]diazepin-11-on
8-Chlor-5-(7-diethylamino-önanthoyl)-5,10-dihydro-11H-dibenzo[b,e] [1,4]diazepin-11-on
8-Chlor-5-(6-N-morpholino-capronyl)-5,10-dihydro-11H-dibenzo [b,e] [1,4]diazepin-11-on
8-Chlor-5-(7-N-morpholino-önanthoyl)-5,10-dihydro-11H-dibenzo [b,e] [1,4]diazepin-11-on
8-Chlor-5,10-dihydro-5-[6-(4-methyl-piperazin-1-yl)capronyl]-11H-dibenzo [b,e] [1,4]diazepin-11-on
5,10-Dihydro-5-[6-(4-methyl-piperazin-1-yl)-capronyl]-11H-dibenzo[b,e] [1,4]diazepin-11-on
5-(6-Diisopropylamino-capronyl)-5,10-dihydro-11H-dibenzo[be] [1,4]diazepin-11-on
5-(6-Ethylamino-capronyl)-5,10-dihydro-11H-dibenzo[b,e]-[1,4]diazepin-11-on
8-Chlor-5-(6-ethylamino-capronyl)-5,10-dihydro-11H-dibenzo[b,e] [1,4]diazepin-11-on
5-(6-Isopropylamino-capronyl)-5,10-dihydro-11H-dibenzo[b,e] [1,4]diazepin-11-on
8-Chlor-5-(6-isopropylamino-capronyl)-5,10-dihydro-11H-dibenzo[b,e] [1,4]diazepin-11-on
5-(6-Cyclohexylamino-capronyl)-5,10-dihydro-11H-dibenzo[b,e] [1,4]diazepin-11-on und
8-Chlor-5-(6-cyclohexylamino-capronyl)-5,10-dihydro-11H-dibenzo[b,e][1,4]diazepin-11-on
sowie ihre Salze.

Eine besondere Ausführungsform der Erfindung betrifft 5-(ω-Aminoacyl)-5,10-dihydro-11H-dibenzon[b,e] [1,4]diazepIn-11-one der allgemeinen Formel VII

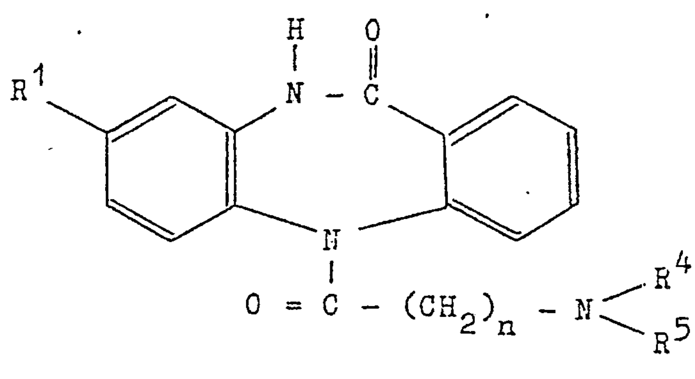

(VII),

worin bedeuten:

$R^1$ Wasserstoff oder Chlor, $R^4$ und $R^5$, die gleich oder verschieden sein können, $C_{1-3}$ -Alkyl oder zusammen mit dem N-Atom, an das sie gebunden sind, einen Morpholino- oder N-Methylpiperazinorest und

n 3, 4, 5 oder 6,

sowie ihre Salze, insbesondere mit physiologisch verträglichen anorganischen und organischen Säuren.

Eine weitere besondere Ausführungsform der Erfindung betrifft 5-(ω-Aminoacyl)-5,10-dihydro-11H-dibenzo[b,e]-[1,4] diazepin-11-one der allgemeinen Formel X

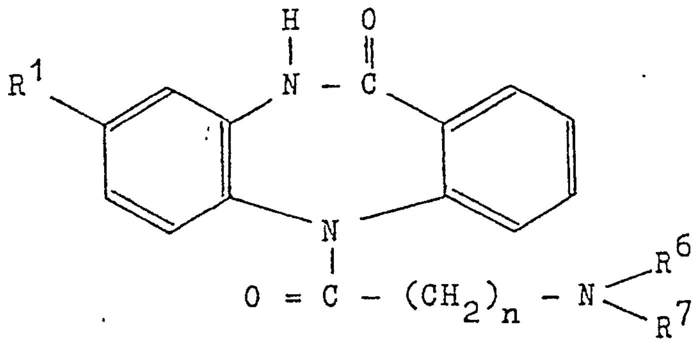

(X),

worin bedeuten:

$R^1$ Wasserstoff oder Chlor,

$R^6$ Wasserstoff,

$R^7$ $C_{1-3}$ -Alkyl oder Cyclohexyl

und

n 3, 4, 5 oder 6,

sowie ihre Salze, insbesondere mit physiologisch verträglichen anorganischen und organischen Säuren.

Das erfindungsgemäße Verfahren zur Herstellung der oben definierten 5-(ω-Aminoacyl)-5,10-dihydro-11-dibenzo[b,e] [1 ,4]diazepin-11-one der allgemeinen Formeln I, VII und X ist gekennzeichnet durch folgende Maßnahmen:

(A1) zur Herstellung der Verbindungen der Formel I

(a) Umsetzung eines 5,10-Dihydro-5-(ω -halogenacyl)-11H-dibenzo [b,e] [1,4] diazepin-11-ons der allgemeinen Formel II,

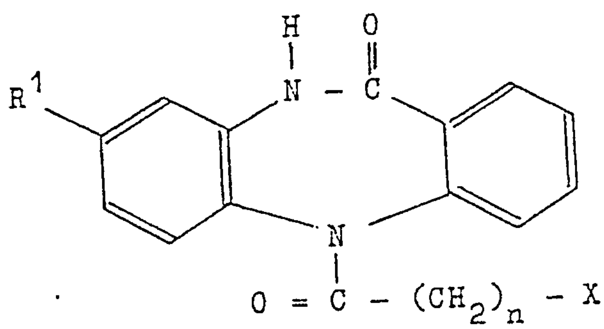

(II),

worin $R^1$ und n dasselbe wie in Formel I und X ein Halogenatom bedeuten, mit einem Amin der allgemeinen Formel III

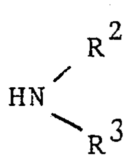

(III)

mit $R^2$ und $R^3$ wie in Formel I oder einem Salz dieses Amins

oder

(b) Umsetzung eines 5,10-Dihydro-11H-dibenzo[b,e] [1,4]diazepin-11-ons der allgemeinen Formel IV

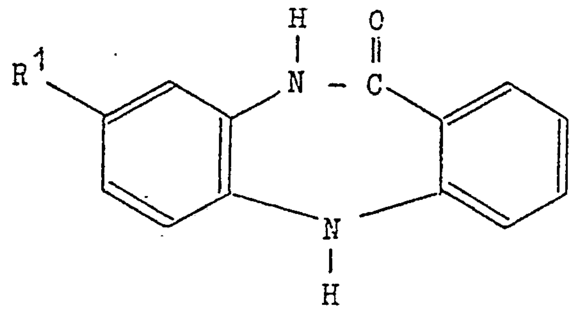

(IV)

mit $R^1$ wie oben
mit einem Aminoacylhalogenid der allgemeinen Formel V,

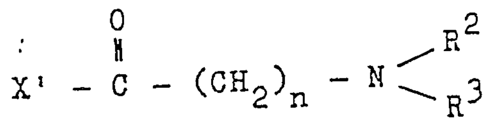

(V),

worin $R^2$, $R^3$ und n dasselbe wie oben
und
X′ ein Halogenatom, vorzugsweise Cl oder Br, bedeuten,
oder einem Salz dieses Aminoacylhalogenids,

(A2) zur Herstellung der Verbindungen der Formel VII

(a) Umsetzung eines 5,10-Dihydro-5-(ω-halogenacyl)-11H-dibenzo[b,e] [1,4]diazepin-11-ons der allgemeinen Formel II
wie oben definiert
mit einem Amin der allgemeinen Formel VIII

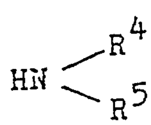

(VIII)

mit $R^4$ und $R^5$ wie in Formel VII oder einem Salz dieses Amins
oder

(b) Umsetzung eines 5,10-Dihydro-11H-dibenzo[b,e] [1,4] diazepin-11-ons der allgemeinen Formel IV wie oben definiert mit einem Aminoacylhalogenid der allgemeinen Formel IX,

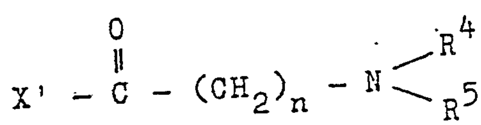

(IX),

worin $R^4$, $R^5$ und n dasselbe wie oben
und
X′ ein Halogenatom, vorzugsweise Cl oder Br,
bedeuten,
oder einem Salz dieses Aminoacylhalogenids,

(A3) zur Herstellung der Verbindungen der Formel X

(a) Umsetzung eines 5,10-Dihydro-5-(ω -halogenacyl)-11H-dibenzo[b,e] [1,4]diazepin-11-ons der allgemeinen Formel II
wie oben definiert
mit einem Amin der allgemeinen Formel XI

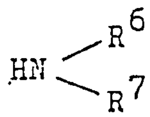

(XI)

mit $R^6$ und $R^7$ wie in Formel X
oder einem Salz dieses Amins
oder
(b) Umsetzung eines 5,10-Dihydro-11H-dibenzo[b,e] [1,4]diazepin-11-ons der allgemeinen Formel IV wie oben definiert
mit einem Aminoacylhalogenid der allgemeinen Formel XII

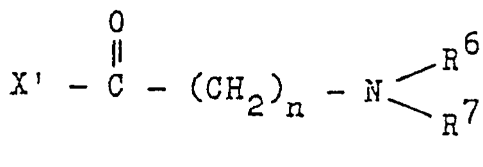

(XII)

worin $R^6$ und $R^7$ dasselbe wie oben
und
X′ ein Halogenatom, vorzugsweise Cl oder Br,
bedeuten,
oder einem Salz dieses Aminoacylhalogenids sowie erwünschtenfalls

(B) Überführung der erhaltenen Verbindungen der Formeln I, VII bzw. X, vorzugsweise mit physiologisch verträglichen anorganischen oder organischen Säuren, in entsprechende Säureadditionssalze und/oder erwünschtenfalls
(C) Freisetzung der entsprechenden Basen der Formeln I, VII bzw. X aus Salzen dieser Verbindungen.

Eine besondere Ausführungsform des Verfahrens nach der Erfindung besteht darin, daß die Herstellung der Verbindungen der allgemeinen Formeln I, VII bzw. X in einem inerten organischen Lösungsmittel durchgeführt wird. Als inerte organische Lösungsmittel eignen sich zum Beispiel Alkohole wie Ethanol, n-Propanol und Isopropanol, Ether wie Dioxan und Tetrahydrofuran, aromatische Kohlenwasserstoffe wie Benzol, Toluol oder Xylol, halogenierte aliphatische oder aromatische Kohlenwasserstoffe wie Chloroform, 1,2-Dichlorethan, Tetrachlorkohlenstoff und Chlorbenzol oder dipolar-aprotische Lösungsmittel wie Dimethylformamid, Acetonitril und Dimethylsulfoxid.

Eine weitere Ausführungsform des erfindungsgemäßen Verfahrens besteht darin, daß als Lösungsmittel ein Überschuß der Verbindungen der Formeln III, VIII bzw. XI verwendet wird.

Bei der Herstellung der Verbindungen der allgemeinen Formeln I, VII bzw. X kann die Temperatur in weiten Grenzen variieren. Eine besondere Ausführungsform der Erfindung besteht jedoch darin, daß die Herstellung bei Temperaturen zwischen Raumtemperatur und 150 °C durchgeführt wird.

Entsprechend der vorliegenden Erfindung kann die Herstellung der Verbindungen der allgemeinen Formeln I, VII bzw. X in Gegenwart eines Säureacceptors durchgeführt werden. Als Säureacceptoren eignen sich beispielsweise Alkalicarbonate, Alkalihydrogencarbonate oder tertiäre organische Amine wie Triethylamin, Pyridin oder N,N-Dimethylanilin.

Die erhaltenen Verbindungen der allgemeinen Formeln I, VII bzw. X können nach an sich bekannten Methoden mit anorganischen oder organischen und insbesondere physiologisch verträglichen Säuren in ihre Säureadditionssalze überführt werden.

Als Beispiele für solche physiologisch verträglichen anorganischen oder organischen Säuren seien genannt: Chlorwasserstoffsäure, Bromwasserstoffsäure, Jodwasserstoffsäure, Schwefelsäure, Phosphorsäure, Essigsäure, Weinsäure, Citronensäure, Fumarsäure, Maleinsäure, Bernsteinsäure und Ascorbinsäure.

Die vorliegende Erfindung umfaßt ferner auch die pharmazeutische Verwendung der Verbindungen der Formeln I, VII bzw. X, insbesondere in Form von pharmazeutischen Zusammensetzungen bzw. Mitteln, die mindestens eine Verbindung der allgemeinen Formeln I, VII bzw. X oder mindestens ein Salz dieser Verbindungen mit physiologisch verträglichen anorganischen oder organischen Säuren als Wirkstoff enthalten.

Nach einer besonderen Ausführungsform der Erfindung sind diese pharmazeutischen Mittel zur Behandlung von Arrhythmien, insbesondere von Bradyarrhythmien, vorgesehen.

Nach einer weiteren Ausführungsform der Erfindung sind diese pharmazeutischen Mittel zur Behandlung von cholinerg induzierten Erkrankungen vorgesehen.

Als pharmazeutische Zubereitungen eignen sich insbesondere Tabletten, Dragees, Kapseln, Lösungen, Ampullen oder Suppositorien mit einem Gehalt an mindestens einer Verbindung der allgemeinen Formeln I, VII bzw. X oder deren Salzen mit physiologisch verträglichen anorganischen oder organischen Säuren, gegebenenfalls im Gemisch mit inerten, pharmazeutisch geeigneten Träger- und/oder Hilfsstoffen. Sie können nach in der pharmazeutischen Praxis allgemein bekannten und üblichen Verfahren hergestellt werden.

Die als Ausgangsverbindungen für die Verfahren (b) eingesetzten 5,10-Dihydro-11H-dibenzo[b,e][1,4]-diazepin-11-one der allgemeinen Formel IV sind bekannt.

Die bei den Verfahren (a) eingesetzten Ausgangsverbindungen der allgemeinen Formel II lassen sich analog literaturbekannten Methoden durch Umsetzung eines 5,10-Dihydro-11H-dibenzo[b,e][1,4]diazepin-11-ons der allgemeinen Formel IV, worin $R^1$ die oben genannte Bedeutung besitzt, mit einem Halogenacylhalogenid der allgemeinen Formel VI herstellen,

$$X' - \overset{\overset{\displaystyle O}{\|}}{C} - (CH_2)_n - X \qquad (VI),$$

worin X, X′ und n die oben genannte Bedeutung besitzen, gegebenenfalls in Gegenwart halogenwasserstoffbindender Mittel wie Alkalicarbonate oder Alkalihydrogencarbonate.

Diese Umsetzung erfolgt vorzugsweise in einem inerten organischen Lösungsmittel bei erhöhten Temperaturen. Als Lösungsmittel können aromatische Kohlenwasserstoffe wie Benzol, Toluol oder Xylol, halogenierte aromatische Kohlenwasserstoffe wie Chlorbenzol, aber auch Ester wie Essigsäureethylester verwendet werden.

Die erfindungsgemäßen neuen 5-(ω-Aminoacyl)-5,10-dihydro11H-dibenzo[b,e] [1,4]diazepin-11-one der allgemeinen Formeln I, VII bzw. X zeigen bei der tierexperimentellen Prüfung eine ausgeprägte antiarrhythmische Wirkung. In verschiedenen pharmakologischen Modellen zur Prüfung der antiarrhythmischen Wirksamkeit erwiesen sich die erfindungsgemäßen Verbindungen als wesentlich stärker wirksam als Lidocain und Chinidin.

So konnte an der $CaCl_2$-Arrhythmie der Ratte (Methodik bei Femmer, K., und Mitarb., Pharmazie 40 , S. 836 (1985)) gegenüber den Vergleichspräparaten Lidocain und Chinidin eine deutlich stärkere antifibrillatorische Wirkung nachgewiesen werden (siehe Tabelle 1).

Die Verbindungen der allgemeinen Formeln I, VII bzw. X zeigen bei intravenöser Anwendung an Ratten eine bessere Verträglichkeit als ihre Referenzpräparate.

Aufgrund der erhöhten Wirkungsstärke und der besseren Verträglichkeit der Verbindungen der allgemeinen Formeln I, VII bzw. X ergibt sich eine wesentliche Vergrößerung der therapeutischen Breite (siehe Tabelle 1, $Q = LD_{50}/ED_{50}$).

Da alle bisher angewandten Antiarrhythmika eine geringe therapeutische Breite besitzen, ist durch die erfindungsgemäßen neuen Verbindungen auch in dieser Hinsicht ein deutlicher Vorteil für die sicherere medizinische Anwendung bei der Behandlung von Herzrhythmusstörungen zu erwarten.

Am Modell der zweistufigen Koronarligatur am Hund nach Harris (Methodik bei Kaverina und Mitarbeiter, Pharmazie 40, S. 845 (1985)) zeigen die Verbindungen der allgemeinen Formeln I, VII bzw. X eine starke und langanhaltende Wirkung (siehe Tabellen 2 und 3).

Bemerkenswert ist, daß die neuen Verbindungen im Gegensatz zu anderen Antiarrhythmika ohne anticholinerge Wirkung, wie Lidocain, und Antiarrhythmika mit anticholinerger Wirkung, wie Chinidin (Mokler, C.M. und C.G. van Arman, J. Pharmacol. exp. Ther., 136 , S. 114 (1962), [1], die an diesem Modell zu einer Abnahme der Gesamtzahl der Herzkontraktionen und der ektopischen Arrhythmie führen, die Gesamtzahl der Herzkontraktionen bei gleichzeitiger Senkung der Ektopierate unbeeinflußt lassen.

Bereits in der 5. Minute nach der intravenösen Injektion der Verbindungen der allgemeinen Formeln I, VII bzw. X in einer Konzentration von 0,005 mmol/kg (ca. 2,0 mg/kg) wird die ektopischeArrhythmie um das 2- bis 3-fache des Basiswertes unterdrückt, während die Gesamtzahl der Herzkontraktionen konstant bleibt. Die Wirkung auf die ektopische Arrhythmie hält 30 bis 60 Minuten lang an.

Tabelle 1

| Antifibrillatorische Wirkung anhand der $CaCl_2$-Arrhythmie der Ratte und der Koronarligatur am Hund sowie orientierende Bestimmung der akuten Toxizität an Ratten im Vergleich zu Standardpräparaten | | | | | |
|---|---|---|---|---|---|
| Verbindung | $CaCl_2$-Arrhythmie Ratte, $ED_{50}$ (mg/kg) | akute Toxizität Ratte $LD_{50}$ i.v., (mg/kg) | Q | Harris Hund | |
| | | | $LD_{50}/ED_{50}$ | eff. Dosis i.v. (mg/kg) | Wirkungsdauer (min.) |
| Beispiel 1 | 0,41 (0,22 - 0,78) | 85 (81 - 90) | 207 | 1,76 | 60 |
| Beispiel 4 | 0,044 (0,016 - 0,12) | 71 (62 - 81) | 1614 | 1,93 | 30 |
| Beispiel 6 | 0,44 (0,25 - 0,76) | 38 (32 - 45) | 86 | 2,07 | 60 |
| Beispiel 9 | 0,16 (0,042 - 0,58) | 41 (37 - 47) | 256 | 2,21 | 120 |
| Lidocain | 3,1 (1,5 - 6,6) | 18 (17 - 21) | 5,8 | 8,0 | 15 |
| Chinidin | 2,7 (2,0 - 3,7) | 48 (47 - 49) | 18 | 10,0 [1] | 15 |

Tabelle 2

| Wirkung auf die Gesamtzahl der Herzkontraktionen an Hunden, 24 Stunden nach zweistufiger Koronarligatur (Harris-Modell) | | | | | | | | | | | |
|---|---|---|---|---|---|---|---|---|---|---|---|
| Verbindung | Dosis (mmol/kg) (mg/kg) | N | Gesamtzahl der Herzkontraktionen/min | | | | | | | | |
| | | | Basis | (min) | 5 | 30 | 60 | 120 | 180 | 300 | 360 |
| Beispiel 1 | 0,005 | 4 | $\bar{x}$ | 164 | 192 | 193 | 177 | 175 | 169 | - | - |
| | 1,76 | | s | 10,1 | 10,4 | 6,3 | 4,3 | 8,1 | 11,2 | | |
| Beispiel 4 | 0,005 | 4 | $\bar{x}$ | 183 | 175 | 180 | 181 | 183 | - | - | - |
| | 1,93 | | s | 13 | 15 | 15,9 | 14,5 | 11,7 | | | |
| Beispiel 6 | 0,005 | 5 | $\bar{x}$ | 167 | 170 | 159 | 162 | 162 | - | - | - |
| | 2,07 | | s | 8,6 | 8,2 | 9,3 | 7,2 | 6,6 | | | |
| Beispiel 9 | 0,005 | 3 | $\bar{x}$ | 156 | 179 | 164 | 168 | 156 | 159 | 163 | 180 |
| | 2,21 | | s | 12,8 | 9,5 | 7,1 | 12,7 | 13,7 | 11,9 | 19,1 | 0,4 |
| Lidocain | 0,02 | 5 | $\bar{x}$ | 146 | 139 | 138 | - | - | - | - | - |
| | 5,4 | | s | 7,3 | 6,7 | 3,8 | | | | | |

Tabelle 3

| Wirkung auf die ektopischen Kontraktionen in Prozent an Hunden, 24 Stunden nach zweistufiger Koronarligatur (Harris-Modell) | | | | | | | | | | | |
|---|---|---|---|---|---|---|---|---|---|---|---|
| Verbindung | Dosis (mmol/kg) (mg/kg) | N | Ektopische Kontraktionen in Prozent/min | | | | | | | | |
| | | | Basis | (min) | 5 | 30 | 60 | 120 | 180 | 300 | 360 |
| Beispiel 1 | 0,005 | 4 | $\bar{x}$ | 99 | 30 | 47 | 54 | 68 | 80 | - | - |
| | 1,76 | | s | 0,75 | 18,1 | 16,6 | 15,7 | 3,4 | 5,5 | | |
| Beispiel 2 | 0,005 | 4 | $\bar{x}$ | 88 | 26 | 24 | 75 | 90 | - | - | - |
| | 1,93 | | s | 5,3 | 16,9 | 6,5 | 7,2 | 2,3 | | | |
| Beispiel 6 | 0,005 | 5 | $\bar{x}$ | 78 | 20 | 4 | 37 | 76 | - | - | - |
| | 2,07 | | s | 7,5 | 10,1 | 3,1 | 14,9 | 6,6 | | | |
| Beispiel 9 | 0,005 | 3 | $\bar{x}$ | 90 | 21 | 38 | 42 | 42 | 73 | 79 | 89 |
| | 2,21 | | s | 2,0 | 5,7 | 15,0 | 23,2 | 14,1 | 9,7 | 4,9 | 2,3 |
| Lidocain | 0,02 | 5 | $\bar{x}$ | 83 | 34 | 78 | - | - | - | - | - |
| | 5,4 | | s | 6,2 | 17,1 | 7,0 | | | | | |

## Legenden:

**Zu Tabelle 1:**

$ED_{50}$ i.v. = Dosis in mg/kg, welche die fibrillatorische Wirkung von $CaCl_2$ an Ratten nach I.v.-Applikation der Testsubstanz um 50 % unterdrückt. Berechnung nach der Probit-Regressionsanalyse mit Vertrauensbereich, p = 0,05 (Werte in Klammern)
$LD_{50}$ i.v. = Letale Dosis in mg/kg bei 50 % der Versuchstiere einer orientierenden Bestimmung der akuten Toxizität an Wistarratten hauseigener Zucht, I.v.-Applikation in die Schwanzvene, 3 - 4 Dosierungen zu je 10 Tieren.

Berechnung nach der Probit-Regressionsanalyse mit Vertrauensbereich, p = 0,05 (Werte in Klammern)

$$Q \quad = \text{Quotient} \quad \frac{LD_{50}\ i.v.}{ED_{50}\ i.v.}$$

zur Ermittlung der therapeutischen Breite
eff. Dosis = effektive Dosis in mg/kg
Wirkungsdauer = Wirkungsdauer in Minuten an Hunden, 24 h nach zweistufiger Koronarligatur

**Zu Tabellen 2 und 3:**

$\bar{x}$ = Mittelwert

s = Standardabweichung ±

Die Erfindung wird im folgenden anhand von Ausführungsbeispielen näher erläutert.

**Beispiel 1**

5-(6-Dimethylaminocapronyl)-5,10-dihydro-11H-dibenzo [b,e]-[1,4] diazepin-11-on•HCl

7,75 g (0,02 mol) 5-(6-Bromcapronyl)-5,10-dihydro-11H-dibenzo[b,e] [1,4]diazepin-11-on werden in 50 ml Dimethylformamid bei Raumtemperatur gelöst und auf 2 °C abgekühlt; nach Zusatz von 10 ml einer Lösung von Dimethylamin in Dimethylformamid (72-%ig, entsprechend 0,16 mol Dimethylamin) wird im geschlossenen Gefäß langsam auf Raumtemperatur und anschließend eine halbe Stunde bei 40 °C Badtemperatur erwärmt. Danach erfolgt eine weitere Zugabe von 10 ml Dimethylaminlösung, und es wird wie oben beschrieben verfahren. Anschließend destilliert man das Dimethylformamid im Vakuum ab, verteilt das verbleibende Öl zwischen 30 ml verdünnter Ammoniaklösung und 50 ml Chloroform und schüttelt die ammoniakalische Phase noch zweimal mit je 20 ml Chloroform und die vereinigten Chloroformphasen dreimal mit je 20 ml 1:1 verdünnter Salzsäure aus.

Die salzsauren Phasen werden mit ca. 10 ml konzentrierter Ammoniaklösung basisch gestellt und zweimal mit je 10 ml Chloroform ausgeschüttelt; die vereinigten Chloroformphasen werden über Natriumsulfat getrocknet.

Nach dem Abtrennen des Natriumsulfats destilliert man das Chloroform ab, versetzt mit 40 ml Aceton und leitet in die Lösung HCl bis pH 1 ein. Anschließend engt man ein, versetzt den Rückstand mit 30 ml Essigsäureethylester und 20 ml Aceton, saugt das Kristallisat nach 12 h ab und kristallisiert aus Isopropanol um.

Die Ausbeute beträgt 4,7 g (61% der Theorie), Schmelzpunkt: 183 °C.

| Analyse für $C_{21}H_{26}N_3O_2Cl$: | berechnet | gefunden |
|---|---|---|
| C (%) | 65,02 | 64,69 |
| H (%) | 6,76 | 6,78 |
| N (%) | 10,83 | 10,73 |
| Cl (%) | 9,14 | 9,29. |

Das eingesetzte Ausgangsprodukt 5-(6-Bromcapronyl)-5,10-dihydro-11H-dibenzo[b,e] [1,4]diazepin-11-on wird wie folgt hergestellt:

14,4 g (0,07 mol) 5,10-Dihydro-11H-dibenzo[b,e] [1,4]-diazepin-11-on werden in (1,00 ml Toluol gelöst, worauf 18,8 g (0,088 mol) Bromcapronsäurechlorid langsam zugetropft werden und das Reaktionsgemisch 5 h am Rückfluß gerührt wird. Anschließend setzt man 2 g Aktivkohle zu, erhitzt nochmals 15 min am Rückfluß, filtriert und läßt kristallisieren. Das Kristallisat wird abgesaugt mit wenig Toluol gewaschen und nach dem Trocknen aus Isopropanol umkristallisiert.

Die Ausbeute beträgt 17,8 g (65,6% der Theorie), Schmelzpunkt: 138 - 139 °C.

| Analyse für $C_{19}H_{19}BrN_2O_2$: | berechnet | gefunden |
|---|---|---|
| C (%) | 58,92 | 59,08 |
| H (%) | 4,94 | 4,94 |
| N (%) | 7,23 | 7,24. |

**Beispiel 2**

**5-(4-Diethylaminobutyryl )-5,10-dihydro-11H-dibenzo[b,e]-[1,4]diazepin-11-on**

3,59 g (0,01 mol) 5-(4-Brombutyryl)-5,10-dihydro-11H-dibenzo[b,e][1,4]diazepin-11-on werden in 25 ml Diethylamin gelöst und 5 h am Rückfluß erhitzt. Anschließend destilliert man das überschüssige Diethylamin ab, versetzt den Rückstand mit 15 ml Wasser und 5 ml wäßriger Ammoniaklösung und extrahiert das Reaktionsprodukt mit Dichlorethan. Die Dichlorethanlösung wird dreimal mit 1:2 verdünnter Salzsäure ausgeschüttelt; die salzsauren Phasen werden mit Ammoniaklösung basisch gestellt und mit Dichlorethan extrahiert. Das Dichlorethan destilliert man ab und kristallisiert den Rückstand aus Toluol um.

Die Ausbeute beträgt 2,95 g (84 % der Theorie), Schmelzpunkt: 166- 167 °C.

| Analyse für $C_{21}H_{25}N_3O_2$: | berechnet | gefunden |
|---|---|---|
| C (%) | 71,77 | 71,56 |
| H (%) | 7,17 | 7,31 |
| N (%) | 11,96 | 11,96. |

Das als Ausgangsprodukt eingesetzte 5-(4-Brombutyryl)-5,10-dihydro-11H-dibenzo [b,e] [1,4] diazepin-11-on wird analog dem in Beispiel 1 beschriebenen Ausgangsprodukt aus 5,10-Dihydro-11H-dibenzo [b,e]-[1,4]diazepin-11-on und 4-Brombutyrylchlorid hergestellt. Ausbeute: 74,3 % der Theorie, Schmelzpunkt: 159 - 161 °C (Toluol).

| Analyse für $C_{17}H_{15}N_2O_2Br$: | berechnet | gefunden |
|---|---|---|
| C (%) | 56,84 | 56,98 |
| H (%) | 4,21 | 4,30 |
| N (%) | 7,80 | 7,72 % |
| Br (%) | 22,24 | 22,02 %. |

## Beispiel 3

### 5-(6-Diethylaminocapronyl)-5,10-dihydro-11H-dibenzo[b,e]-[1,4]diazepin-11-on

7,75 g (0,02 mol) 5-(6-Bromcapronyl)-5,10-dihydro-11H-dibenzo[b,e] [1,4]diazepin-11-on werden in 50 ml Diethylamin 5 h am Rückfluß erhitzt. Das Reaktionsgemisch engt man ein und versetzt den Rückstand mit 30 ml Wasser und 10 ml konzentrierter Ammoniaklösung. Anschließend schüttelt man dreimal mit Benzol aus. Die vereinigten Benzolphasen extrahiert man dreimal mit 1:2 verdünnter Salzsäure, stellt die salzsauren Phasen mit konzentrierter Ammoniaklösung basisch und schüttelt das sich abscheidende Öl dreimal mit Benzol aus. Das Benzol wird abdestilliert und der ölige Rückstand (7 g) unter Erwärmen in 30 ml Toluol gelöst. Nach dem Abkühlen fällt ein Niederschlag aus, den man abfiltriert und mit Toluol und Ether wäscht. Die Ausbeute beträgt 5 g (65,9 % der Theorie), Schmelzpunkt: 104 - 105 °C.

| Analyse für $C_{23}H_{29}N_3O_2$: | berechnet | gefunden |
|---|---|---|
| C (%) | 72,79 | 72,63 |
| H (%) | 7,70 | 7,68 |
| N (%) | 11,07 | 11,20. |

Zur Herstellung des 5-(6-Diethylaminocapronyl)-5,10-dihydro-11H-dibenzo [b,e] [1,4] diazepin-11-on-hydrochlorids wird der o.g. ölige Rückstand (7 g) in 50 ml heißem Isopropanol gelöst und in die auf Raumtemperatur abgekühlte Lösung HCl-Gas bis pH 1 eingeleitet. Die Reaktionslösung engt man dann im

Vakuum zur Trockne ein, gibt nochmals 50 ml Isopropanol zu, engt wiederum ein und kristallisiert aus 50 ml Acetonitril unter Zusatz von Aktivkohle um.

Die Ausbeute beträgt 3,8 g (45,7% der Theorie, bezogen auf 5-(6-Bromcapronyl)-5,10-dihydro-11H-dibenzo[b,e][1,4] diazepin-11-on), Schmelzpunkt: 179 - 183 °C.

| Analyse für $C_{23}H_{30}N_3O_2Cl$: | berechnet | gefunden |
|---|---|---|
| C (%) | 66,41 | 66,23 |
| H (%) | 7,27 | 6,99 |
| N (%) | 10,10 | 10,18 |
| Cl (%) | 8,52 | 8,43. |

**Beispiel 4**

**8-Chlor-5-(4-diethylaminobutyryl )-5,10-dihydro-11H-dibenzo[b,e] [1,4]diazepin-11-on**

3,94 g (0,01 mol 5-(4-Brombutyryl)-8-chlor-5,10-dihydro-11H-dibenzo [b,e] [1 ,4] diazepin-11-on werden in 25 ml Diethylamin unter Rühren 5 h am Rückfluß erhitzt; der Überschuß an Diethylamin wird abdestilliert und der Rückstand mit 15 ml Wasser und 5 ml konzentrierter Ammoniaklösung versetzt. Man extrahiert das Gemisch dann dreimal mit Chloroform, schüttelt die vereinigten chloroformischen Phasen dreimal mit 1:2 verdünnter Salzsäure aus, stellt mit konzentrierter Ammoniaklösung basisch und extrahiert das sich abscheidende Öl dreimal mit Chloroform. Anschließend wird das Chloroform abdestilliert und der Rückstand mit etwas trockenem Benzol verrührt, wobei das Rohprodukt kristallisiert. Man kristallisiert aus Toluol um. Die Ausbeute beträgt 2,85 g (73,8 % der Theorie), Schmelzpunkt: 163 - 164 °C.

| Analyse für $C_{21}H_{24}N_3O_2Cl$: | berechnet | gefunden |
|---|---|---|
| C (%) | 65,36 | 65,53 |
| H (%) | 6,27 | 6,41 |
| Cl (%) | 9,19 | 9,01. |

Das als Ausgangsprodukt eingesetzte 5-(5-Brombutyryl)-8-chlor-5,10-dihydro-11H-dibenzo [b,e] [1,4]-diazepin-11-on wird analog dem in Beispiel 1 beschriebenen Ausgangsprodukt aus 8-Chlor-5,10-dihydro-11H-dibenzo [b,e] [1,4] diazepin-11-on und 4-Brombutyrylchlorid hergestellt.

Ausbeute: 83,1% der Theorie, Schmelzpunkt: 176 - 177 °C.

| Analyse für $C_{17}H_{14}N_2O_2BrCl$: | berechnet | gefunden |
|---|---|---|
| C (%) | 51,87 | 51,77 |
| H (%) | 3,58 | 3,65. |

**Beispiel 5**

**8-Chlor-5-(5-diethylaminovaleryl)-5,10-dihydro-11H-dibenzo[b,e] [1,4]diazepin-11-on**

4,07 g (0,01 mol) 5-(5-Bromvaleryl)-8-chlor-5,10-dihydro-11H-dibenzo [b,e][1,4] diazepin-11-on werden in 25 ml Diethylamin 5 h am Rückfluß erhitzt, worauf der Diethylaminüberschuß anschließend abdestilliert und der Rückstand mit 15 ml Wasser und 5 ml konzentrierter Ammoniaklösung versetzt wird. Man extrahiert die ammoniakalische Phase mit Benzol, schüttelt die Benzolphase zweimal mit 1:2 verdünnter Salzsäure aus, stellt mit konzentrierter Ammoniak lösung basisch und extrahiert mit Benzol. Anschließend wird das Benzolabdestilliert und der Rückstand aus Toluol umkristallisiert.

Die Ausbeute beträgt 3,25 g (81,3% der Theorie), Schmelzpunkt: 159 - 160 °C.

| Analyse für $C_{22}H_{26}N_3O_2Cl$: | berechnet | gefunden |
|---|---|---|
| C (%) | 66,07 | 66,00 |
| H (%) | 6,55 | 6,63 |
| Cl (%) | 8,86 | 8,65. |

Das als Ausgangsprodukt eingesetzte 5-(5-Bromvaleryl)-8-chlor-5,10-dihydro-11H-dibenzo [b,e] [,1,4]-diazepin-11-on wird analog dem in Beispiel 1 beschriebenen Ausgangsprodukt aus 8-Chlor-5,10-dihydro-11H-dibenzo[b,e] [1,4]diazepin-11-on und 5-Bromvalerylchlorid hergestellt.

Ausbeute:87% der Theorie, Schmelzpunkt:

| Analyse für $C_{18}H_{16}N_2O_2BrCl$: | berechnet | gefunden |
|---|---|---|
| C (%) | 53,03 | 53,26 |
| H (%) | 3,96 | 4,02 |
| N (%) | 6,87 | 7,14. |

**Beispiel 6**

**8-Chlor-5-(6-diethylaminocapronyl)-5,10-dihydro-11H-dibenzo[be] [1,4]diazepin-11-on**

4,21 g (0,01 mol) 5-(6-Bromcapronyl)-8-chlor-5,10-dihydro-11H-dibenzo [b,e] [1,4]diazepin-11-on werden in 25 ml Diethylamin 5 h am Rückfluß erhitzt. Anschließend destilliert man das überschüssige Diethylamin ab, gibt 15 ml Wasser und 5 ml konzentrierte Ammoniaklösung zu und extrahiert dreimal mit Benzol. Die vereinigten Benzolphasen werden dreimal mit 1:2 verdünnter Salzsäure ausgeschüttelt, worauf die vereinigten salzsauren Phasen mit konzentrierter Ammoniaklösung basisch gestellt werden und das sich abscheidende Öl dreimal mit Benzol extrahiert wird. Die benzolische Phase wäscht man mit Wasser und engt sie ein. Der Rückstand wird aus wenig Toluol umkristallisiert.

Die Ausbeute beträgt 2,65 g (64,1% der Theorie), Schmelzpunkt: 136 - 138 °C.

| Analyse für $C_{23}H_{28}N_3O_2Cl$: | berechnet | gefunden |
|---|---|---|
| C (%) | 66,74 | 66,72 |
| H (%) | 6,82 | 7,02 |
| N (%) | 10,15 | 10,08 |
| Cl (%) | 8,55 | 8,54. |

Das als Ausgangsprodukt eingesetzte 5-(6-Bromcapronyl)-8-chlor-5,10-dihydro-11H-dibenzo [b,e] [1,4] diazepin-11-on wird analog dem in Beispiel 1 beschriebenen Ausgangsprodukt aus 8-Chlor-5,10-dihydro-11H-dibenzo[b,e] [1,4]diazepin-11-on und 6-Bromcapronylchlorid hergestellt.

Ausbeute: 76,6% der Theorie, Schmelzpunkt: 170 - 171 °C (Toluol).

| Analyse für $C_{15}H_{18}N_2O_2BrCl$: | berechnet | gefunden |
|---|---|---|
| C (%)<br>H (%) | 54,11<br>4,30 | 54,01<br>4,26. |

**Beispiel 7**

**8-Chlor-5-(7-diethylaminoönanthoyl)-5,10-dihydro-11H-dibenzo[b,e] [1,4)diazepin-11-on**

4,35 g (0,05 mol) 5-(7-Bromönanthoyl)-8-chlor-5,10-dihydro-11H-dibenzo [b,e] [1,4] diazpein-11-on werden in 25 ml Diethylamin 5 h am Rückfluß erhitzt; anschließend wird der Überschuß an Diethylamin abdestilliert. Den Rückstand versetzt man mit 15 ml Wasser und 5 ml konzentrierter Ammoniaklösung und extrahiert, dreimal mit Benzol; die vereinigten Benzolphasen werden dreimal mit 1:2 verdünnter Salzsäure extrahiert: wonach die salzsauren Phasen mit konzentrierter Ammoniaklösung basisch gestellt werden. Anschließend wird dreimal mit Benzol ausgeschüttelt und das Benzol abdestilliert; der Rückstand wird unter Erwärmen in Toluol gelöst und bis zur Kristallisation stehengelassen. Das Kristallisat saugt man ab und wäscht nacheinander mit Toluol und Ether. Die Ausbeute beträgt 2,8 g (66,6% der Theorie), Schmelzpunkt: 145 - 146 °C.

| Analyse für $C_{24}H_{30}N_3O_2Cl$: | berechnet | gefunden |
|---|---|---|
| C (%)<br>H (%)<br>N (%)<br>Cl (%) | 67,35<br>7,06<br>9,81<br>8,22 | 67,30<br>7,08<br>9,77<br>8,17 |

Das als Ausgangsprodukt eingesetzte 5-(7-Bromönanthoyl)-8-chlor-5,10-dihydro-11H-dibenzo [b,e] [1,4] diazepin-11-on wird analog dem in Beispiel 1 beschriebenen Ausgangsprodukt aus 8-Chlor-5,10-dihydro-11H-dibenzo [b,e] [1,4]diazepin-11-on und 7-Bromönanthoylchlorid hergestellt.
Ausbeute: 71,5% der Theorie, Schmelzpunkt: 159 - 161 °C (Toluol).

| Analyse für $C_{20}H_{20}N_2O_2BrCl$: | berechnet | gefunden |
|---|---|---|
| C (%)<br>H (%)<br>N (%) | 55,13<br>4,63<br>6,43 | 55,02<br>4,68<br>6,40. |

**Beispiel 8**

**8-Chlor-5-(6-N-morpholinocapronyl)-5,10-dihydro-11H-dibenzo[b,e] [1,4]diazepin-11-on**

5,6 g (0,015 mol) 5-(6-Bromcapronyl)-8-chlor-5,10-dihydro-11H-dibenzo [b,e] [1 ,4]diazepin-11-on und 5,23 g (0,06 mol) Morpholin werden in 50 ml Toluol 5 h am Rückfluß gerührt. Nach dem Abkühlen extrahiert

man das gewünschte Produkt mit 1:2 verdünnter Salzsäure aus der toluolischen Phase, stellt mit konzentrierter Ammoniaklösung basisch und schüttelt mit Benzol aus. Nach dem Waschen der Benzolphase mit Wasser wird das Benzol abdestilliert. Den öligen Rückstand löst man unter Erwärmen in 120 ml Toluol, läßt abkühlen, saugt den Niederschlag ab, wäscht nacheinander mit Toluol und Ether und läßt trocknen.

Die Ausbeute beträgt 3,7 g (59,6 % der Theorie), Schmelzpunkt: 135 - 136 °C.

| Analyse für $C_{23}H_{26}N_3O_3Cl$: | berechnet | gefunden |
|---|---|---|
| C (%) | 64,55 | 64,40 |
| H (%) | 6,12 | 6,11 |
| N (%) | 9,82 | 9,90 |
| Cl (%) | 8,28 | 8,23. |

**Beispiel 9**

**8-Chlor-5-(7-N-morpholinoönanthoyl)-5,10-dihydro-11H-dibenzo [b,e] [1,4]diazepin-11-on**

3,27 g (0,0075 mol) 5-(7-Bromönanthoyl)-8-chlor-5,10-dihydro-11H-dibenzo [b,e] [1,4] diazepin-11-on und 2 g (0,0225 mol) Morpholin werden in 25 ml Toluol 5 h am Rückfluß gerührt. Nach dem Abkühlen extrahiert man die Reaktionslösung mit 1:2 verdünnter Salzsäure, stellt mit konzentrierter Ammoniaklösung basisch, extrahiert das gewünschte Produkt dreimal mit Dichlorethan, wäscht die Dichlorethanphase mit Wasser und destilliert das Dichlorethan ab. Der Rückstand wird aus Heptan-Toluol (1:1) umkristallisiert; das Kristallisat wird abgesaugt, mit Ether gewaschen und getrocknet.

Die Ausbeute beträgt 2g (60,7% der Theorie), Schmelzpunkt: 102-103 °C.

| Analyse für $C_{24}H_{28}N_3O_3Cl$: | berechnet | gefunden |
|---|---|---|
| C (%) | 65,22 | 65,31 |
| H (%) | 6,39 | 6,32 |
| N (%) | 9,51 | 9,40 |
| Cl (%) | 8,02 | 7,77. |

**Beispiel 10**

**8-Chlor-5,10-dihydro-5-6-(4-methyl-piperazin-1-yl)capronyl -11H-dibenzo[b,e][1,4]diazepin-11-on**

8,44 g (0,02 mol) 5-(6-Bromcapronyl)-8-chlor-5,10-dihydro-11H-dibenzo [b,e] [1,4] diazepin-11-on, 4,21 g (0,042 mol) N-Methylpiperazin und 50 ml Toluol werden 2 h am Rückfluß gerührt. Anschließend schüttelt man die toluolische Lösung mit 20 ml Wasser aus. Die Toluolphase wird zweimal mit 10 ml 1:1 verdünnter Salzsäure extrahiert. Die vereinigten salzsauren Phasen werden mit konzentrierter Ammoniaklösung basisch gestellt und einmal mit 30 ml und zweimal mit 10 ml Chloroform ausgeschüttelt; wonach die Chloroformphasen eingeengt werden. Zum Rückstand gibt man zweimal 50 ml Isopropanol und engt jedesmal wieder ein. Anschließend gibt man 10 ml Toluol zu, erwärmt leicht und saugt das Kristallisat ab.

Die Ausbeute beträgt 6 g (68% der Theorie), Schmelzpunkt: 142 - 147 °C.

| Analyse für $C_{24}H_{29}N_4O_2Cl$: | berechnet | gefunden |
|---|---|---|
| C (%) | 65,37 | 65,47 |
| H (%) | 6,68 | 6,67 |
| N (%) | 12,71 | 12,31 |
| Cl (%) | 8,04 | 7,98. |

**Beispiel 11**

**5,10-Dihydro-[6-(4-methyl-piperazin-1-yl)-capronyl]-11H-dibenzo[b,e][1,4]diazepin-11-on**

5,8 g (0,015 mol) 5-(6-Bromcapronyl)-5,10-dihydro-11H-dibenzo [b,e][1,4]diazepin-11-on, 3,2 g (0,032 mol) N-Methylpiperazin und 40 ml Toluol werden 4 h am Rückfluß gerührt. Anschließend engt man die Reaktionslösung im Vakuum ein, versetzt mit 50 ml Chloroform, schüttelt die Chloroformphase zweimal mit je 20 ml 1:1 verdünnter Salzsäure aus, versetzt die vereinigten salzsauren Phasen mit 20 ml Chloroform, stellt mit konzentrierter Ammoniaklösung basisch, schüttelt aus und trennt die Chloroformphase ab.

Die ammoniakalische Phase wird noch zweimal mit je 20 ml Chloroform ausgeschüttelt; die vereinigten Chloroformphasen werden über $Na_2SO_4$ getrocknet und eingeengt. Den öligen Rückstand kocht man mit 20 ml Isopropanol auf, engt ein und kristallisiert ihn unter Erwärmen mit 20 ml Toluol.

Die Ausbeute beträgt 4,7 g (77% der Theorie); Schmelzpunkt: 129 °C (Zersetzung).

| Analyse für $C_{24}H_{30}N_4O_2$: | berechnet | gefunden |
|---|---|---|
| C (%) | 70,91 | 70,52 |
| H (%) | 7,44 | 7,37 |
| N (%) | 13,78 | 13,46. |

**Beispiel 12**

**8-Chlor-5-(6-ethylaminocapronyl)-5,10-dihydro-11H-dibenzo [b,e] [1,4]diazepin-11-on-HCl**

8)44 g (0,02 mol) 5-(6-Bromcapronyl)-8-chlor-5,10-dihydro-11H-dibenzo[b,e] [1,4]diazepin-11-on werden in 50 ml Dimethylformamid gelöst, bei 2 °C mit 10 ml. Ethylaminlösung (0,17 mol, ca. 75-%ig in Dimethylformamid) versetzt und 0,5 h im geschlossenen Gefäß auf 50 °C erwärmt. Dann wird nochmals bei 2 °C mit 5 ml der obigen Ethylaminlösung versetzt, wiederum 0,5 h bei 50 °C im geschlossenen Gefäß erwärmt, mit 10 % Aktivkohle in der Hitze gerührt und im Vakuum zur Trockne eingeengt.

Den Rückstand versetzt man mit 50 ml Chloroform, engt zur Trockne ein, nimmt in 50 ml Isopropanol, wenn notwendig unter Zusatz von 20 bis 40 % Wasser, auf, und leitet HCl-Gas bis pH 1 ein.

Die saure Lösung wird im Vakuum zur Trockne eingeengt, nochmals mit Isopropanol versetzt, wiederum zur Trockne eingeengt, aus Isopropanol mit Aktivkohle umkristallisiert (Fp. Mettler: 187 - 191 °C) und nochmals mit 30 ml Acetonitril ausgekocht.

Die Ausbeute beträgt 3,0 g (35,4 % der Theorie), Schmelzpunkt: 190 - 195 °C (Zersetzung).

| Analyse für $C_{21}H_{25}N_3O_2Cl_2$: | berechnet | gefunden |
|---|---|---|
| C (%) | 59,72 | 59,40 |
| H (%) | 5,97 | 5,94 |
| N (%) | 9,95 | 9,96 |
| Cl (%) | 16,79 | 16,97. |

Beispiel 13

**5-(6-Ethylaminocapronyl)-5,10-dihydro-11H-dibenzo [b,e]-[1,4] diazepin-11-on • HCl**

7,8 g (0,02 mol) 5-(6-Bromcapronyl)-5,10-dihydro-11H-dibenzo [b,e] [1 ,4]diazepin-11-on werden in 50 ml Dimethylformamid gelöst, bei 2 °C mit 10 ml Ethylaminlösung (0,17 mol, ca. 75-%ig in Dimethylformamid) versetzt, 0,5 h bei 50 °C im geschlossenen Gefäß erwärmt, nochmals mit 10 ml der obigen Ethylaminlösung bei 2 °C versetzt und 1 h bei 50 °C belassen.

Die Reaktionslösung wird anschließend mit 10% (bezogen auf eingesetztes Produkt) Aktivkohle kalt ausgerührt und im Vakuum zur Trockne eingeengt. Den Rückstand löst man in 30 ml Chloroform, schüttelt zweimal mit 20 ml 1:1 verdünnter HCl aus, gibt zu den vereinigten salzsauren Phasen 20 ml Chloroform und stellt mit konzentrierter Ammoniaklösung basisch. Die Chloroformphase wird nach dem Ausschütteln abgetrennt; die ammoniakalische Phase wird noch zweimal mit 20 ml Chloroform ausgeschüttelt. Die vereinigten Chloroformphasen werden über $Na_2SO_4$ getrocknet, wobei etwas Aktivkohle zugesetzt wird. Nach dem Filtrieren engt man die Chloroformphase ein, versetzt den öligen Rückstand mit 20 ml Isopropanol und 6 ml Wasser, leitet HCl-Gas bis pH 1 ein, engt im Vakuum zur Trockne ein, gibt nochmals 50 ml Isopropanol zu, engt nochmals zur Trockne ein und kocht mit 25 ml Acetonitril aus.

Die Ausbeute beträgt 5,1 g (65,4% der Theorie), Schmelzpunkt: 189,5 - 196,5 °C (Zersetzung).

| Analyse für $C_{21}H_{26}N_3O_2Cl$: | berechnet | gefunden |
|---|---|---|
| C (%) | 65,02 | 65,23 |
| H (%) | 6,76 | 6,54 |
| N (%) | 10,83 | 10,89 |
| Cl (%) | 9,14 | 8,91. |

Beispiel 14

**5-(6-Diisopropylaminocapronyl )-5,10-dihydro-11H-dibenzo[b,e] [1,4]diazepin-11-on**

5,8 g (0,015 mol) 5-(6-Bromcapronyl)-5,10-dihydro-11H-dibenzo[b,e] [1,4] diazepin-11-on werden mit 20 ml Diisopropylamin 21 h am Rückfluß erhitzt. Dann engt man das Reaktionsgemisch ein, nimmt den Rückstand in 50 ml Chloroform auf und extrahiert zweimal mit 1:1 verdünnter Ammoniaklösung und anschließend zweimal mit 20 ml und einmal mit 10 ml konzentrierter HCl. Die vereinigten salzsauren Phasen werden mit 20 ml Chloroform ausgeschüttelt, mit konzentrierter Ammoniaklösung stark basisch gestellt und dreimal mit 30 ml Chloroform extrahiert. Die vereinigten Chloroformphasen schüttelt man zweimal mit 20 ml Wasser aus und engt sie zur Trockne ein. Der Rückstand wird in der Hitze in Toluol gelöst und kristallisiert nach längerem Stehen.

Die Ausbeute beträgt 1,7 g (25,6% der Theorie), Schmelzpunkt: 100 - 106 °C (Zersetzung).

| Analyse für $C_{25}H_{32}N_3O_2$: | berechnet | gefunden |
|---|---|---|
| C (%) | 73,68 | 73,66 |
| H (%) | 8,16 | 7,93 |
| N (%) | 10,31 | 10,40. |

## Beispiel 15

**5-(6-Diethylaminocapronyl)-5,10-dihydro-11H-dibenzo[b,e] [1,4]diazepin-11-on**

10 g (0,029 mol) 5-(6-Chlorcapronyl)-5,10-dihydro-11H-dibenzo[b,e] [1,4]diazepin-11-on werden in 100 ml Dimethyl-formamid gelöst, mit 50 ml (0,48 mol) Diethylamin versetzt und 33 h bei 65 °C Badtemperatur gerührt. Anschließend engt man das Reaktionsgemisch zur Trockne ein. Den Rückstand verteilt man unter Erwärmen zwischen 120 ml Toluol, 70 ml Wasser und 40 ml konzentrierter Ammoniaklösung, trennt das Toluol ab, schüttelt die wäßrige Phase noch zweimal mit je 50 ml Toluol aus und extrahiert die vereinigten Toluolphasen dreimal mit je 50 ml 1:1 verdünnter Salzsäure.

Die vereinigten salzsauren Phasen werden mit konzentrierter Ammoniaklösung stark basisch gestellt und dreimal mit 50 ml Toluol ausgeschüttelt; die vereinigten Toluolphasen werden zweimal mit 50 ml Wasser ausgeschüttelt.

Die Toluolphasen engt man im Vakuum ein, versetzt nochmals mit 50 ml Toluol und engt wieder ein. Der Rückstand wird zweimal aus Toluol unter Zusatz von Aktivkohle umkristallisiert. Bei der Kristallisation der Verbindung werden 10 ml Cyclohexan zugegeben.

Die Ausbeute beträgt 3,1 g (27,2 der Theorie), Schmelzpunkt: 98 - 100 °C.

| Analyse für $C_{23}H_{29}N_3O_2$: | berechnet | gefunden |
|---|---|---|
| C (%) | 72,79 | 72,62 |
| H (%) | 7,70 | 7,62 |
| N (%) | 11,07 | 11,08. |

## Beispiel 16

**8-Chlor-5-(6-cyclohexylaminocapronyl)-5,10-dihydro-11H-dibenzo[b,e] [1,4]diazepin-11-on**

8,44 g (0,02 mol) 5-(6-Bromcapronyl)-8-chlor-J,10-dihydro-11H-dibenzo [b,e] [1,4] diazepin-11-on und 4 g (0,04 mol) Cyclohexylamin werden in 50 ml Toluol 5 h am Rückfluß erhitzt. Anschließend versetzt man das Reaktionsgemisch mit 25 ml Toluol und 50 ml Wasser und rührt so lange, bis sich die bei der Reaktion gebildete ölartige Schicht vollständig aufgelöst hat. Nach dem Abtrennen der wäßrigen Phase wird die Toluolphase noch dreimal mit Wasser ausgeschüttelt und das Toluol abdestilliert. Den Rückstand löst man in 90 ml Aceton, filtriert und versetzt die Acetonlösung mit 3 ml konzentrierter Salzsäure. Das ausgefällte Öl löst man durch Rühren, und nach einiger Zeit fällt aus der Lösung ein kristalliner Niederschlag aus, den man aus absolutem Ethanol umkristallisiert.

Die Ausbeute beträgt 6,3 g (64,9% der Theorie), Schmelzpunkt: 174 - 175 °C.

| Analyse für $C_{25}H_{32}N_3O_{2,5}Cl_2$: | berechnet | gefunden |
|---|---|---|
| C (%) | 61,85 | 61,56 |
| H (%) | 6,64 | 6,57 |
| N (%) | 8,65 | 8,66 |
| Cl (%) | 14,61 | 14,67. |

## Beispiel 17

**5-(6-Cyclohexylaminocapronyl)-5,10-dihydro-11H-dibenzo[b,e] [1,4]diazepin-11-on**

3,87 g (0,01 mol) 5-(6-Bromcapronyl)-5,10-dihydro-11H-dibenzo[b,e] [1,4]diazepin-11-on und 2,5 g (0,025 mol) Cyclohexylamin werden in 25 ml Toluol 5 h am Rückfluß erhitzt. Anschließend setzt man dem Reaktionsgemisch 2,3 ml Bromwasserstoffsäure (48-%ig) und 20 ml Wasser zu. Die untere Schicht wird von der oberen Schicht getrennt, mit 30 ml Aceton versetzt und 15 min am Rückfluß erhitzt. Man filtriert den Rückstand und versetzt ihn nach dem Waschen mit Wasser mit 50 ml Aceton. Die erhaltene Suspension wird 1 h am Rückfluß erhitzt; der Rückstand wird filtriert und mit etwas Aceton gewaschen. Nach dem Trocknen beträgt die Ausbeute 2,6 g (52,5 % der Theorie), Schmelzpunkt: 250 -251 °C.

| Analyse für $C_{25}H_{33}N_3O_{2,5}Br$: | berechnet | gefunden |
|---|---|---|
| C (%) | 60,61 | 60,92 |
| H (%) | 6,71 | 6,63 |
| N (%) | 8,48 | 8,65 |
| Br (%) | 16,43 | 16,00. |

## Ansprüche

1. 5-(ω-Aminoacyl)-5,10-dihydro-11H-dibenzo[b,e][1,4]-diazepin-11-one der allgemeinen Formel I

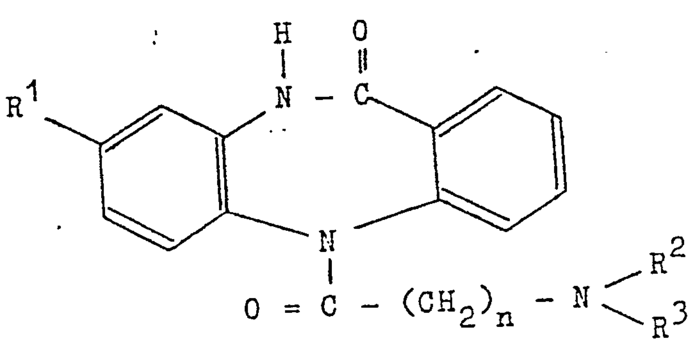

(I),

worin bedeuten:
$R^1$ Wasserstoff oder Chlor,
$R^2$ und $R^3$, die gleich oder verschieden sein können, Wasserstoff, geradkettiges oder verzweigtes $C_{1-3}$-Alkyl oder zusammen mit dem N-Atom, an das sie gebunden sind, einen Morpholino- oder N-Methylpiperazinorest

und
n 3, 4, 5 oder 6,
wobei, wenn $R^2$ Wasserstoff ist, $R^3$ auch Cyclohexyl bedeuten kann,
und
ihre Salze, insbesondere mit physiologisch verträglichen anorganischen und organischen Säuren.
2. 5-(ω-Aminoacyl)-5,10-dihydro-11H-dibenzo[b,e] [1,4]-diazepin-11-one der allgemeinen Formel VII

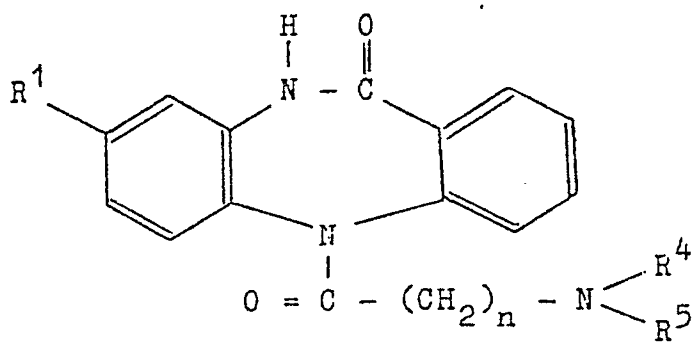

(VII),

worin bedeuten:
$R^1$ Wasserstoff oder Chlor,
$R^4$ und $R^5$, die gleich oder verschieden sein können, $C_{1-3}$-Alkyl oder zusammen mit dem N-Atom, an das sie gebunden sind, einen Morpholino- oder N-Methylpiperazinorest
und
n 3, 4, 5 oder 6,
sowie ihre Salze, insbesondere mit physiologisch verträglichen anorganischen und organischen Säuren.
3. 5-(ω-Aminoacyl)-5,10-dihydro-11H-dibenzo[b,e] [1,4]-diazepin-11-one der allgemeinen Formel X

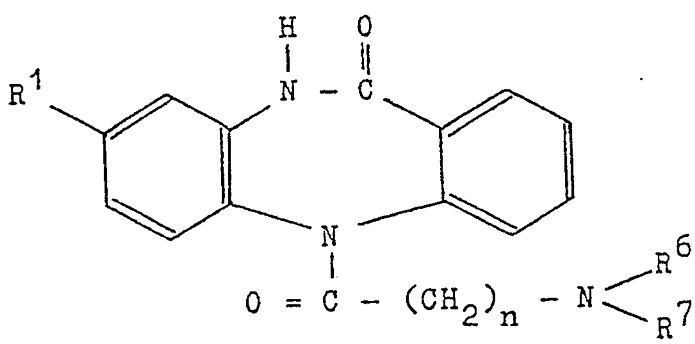

(X),

worin bedeuten:
$R^1$ Wasserstoff oder Chlor,
$R^6$ Wasserstoff,
$R^7$ $C_{1-3}$ -Alkyl oder Cyclohexyl und n 3, 4, 5 oder 6,
sowie ihre Salze, insbesondere mit physiologisch verträglichen anorganischen und organischen Säuren.
4. 5-(6-Dimethylamino-capronyl)-5,10-dihydro-11H-dibenzo-[b,e] [1,4] diazepin-11-on
5-(4-Diethylamino-butyryl)-5,10-dihydro-11H-dibenzo-[b,e] [1,4]diazepin-11-on
5-(6-Diethylamino-capronyl)-5,10-dihydro-11H-dibenzo[b,e] [1,4]diazepin-11-on
8-Chlor-5-(4-diethylamino-butyryl)-5,10-dihydro-11H-dibenzo[b,e] [1,4] diazepin-11-on
8-Chlor-5-(5-diethylamino-valeryl)-5,10-dihydro-11H-dibenzo[b,e] [1,4]diazepin-11-on
8-Chlor-5-(6-diethylamino-capronyl)-5,10-dihydro-11H-dibenzo[b,e] [1,4] diazepin-11-on
8-Chlor-5-(7-diethylamino-önanthoyl)-5,10-dihydro-11H-dibenzo[b,e] [1,4) diazepin-11-on
8-Chlor-5-(6-N-morpholino-capronyl)-5,10-dihydro-11H-dibenzo [b,e] [1,4]diazepin-11-on
8-Chlor-5-(7-N-morpholino-önanthoyl)-5,10-dihydro-11H-dibenzo[b,e] [1,4]diazepin-11-on
8-Chlor-5,10-dihydro-5-[6-(4-methyl-piperazin-1-yl)capronyl]-11H-dibenzo[b,e] [1,4]diazepin-11-on
5,10-Dihydro-5-[6-(4-methyl-piperazin-1-yl)-capronyl]-11H-dibenzo[b,e] [1,4]diazepin-11-on

5-(6-Diisopropylamino-capronyl)-5,10-dihydro-11H-dibenzo[b,e] [1,4]diazepin-11-on
5-(6-Ethylamino-capronyl)-5,10-dihydro-11H-dibenzo[b,e]-[1,4] diazepin-11-on
8-Chlor-5-(6-ethylamino-capronyl)-5,10-dihydro-11H-dibenzo[b,e] [1,4]diazepin-11-on
5-(6-Isopropylamino-capronyl)-5,10-dihydro-11H-dibenzo[b,e] [1,4]diazepin-11-on
8-Chlor-5-(6-isopropylamino-capronyl)-5,10-dihydro-11H-dibenzo [b,e] [1,4]diazepin-11-on
5-(6-Cyclohexylamino-capronyl)-5,10-dihydro-11H-dibenzo[b,e] [1,4]diazepin-11-on und
8-Chlor-5-(6-cyclohexylamino-capronyl)-5,10-dihydro-11H-dibenzo[b,e] [1,4]diazepin-11-on
sowie die Salze dieser Verbindungen, insbesondere mit physiologisch verträglichen anorganischen und organischen Säuren.

5. Verfahren zur Herstellung der 5-(ω-Aminoacyl)-5,10-dihydro-11H-dibenzo[b,e] [1,4]diazepin-11-one der allgemeinen Formeln I, VII und X nach den Ansprüchen 1 bis 4,
gekennzeichnet durch folgende Maßnahmen:
(A1) zur Herstellung der Verbindungen der Formel I
(a) Umsetzung eines 5,10-Dihydro-5-(ω-halogenacyl)-11H-dibenzo[b,e][1,4]diazepin-11-ons der allgemeinen Formel II,

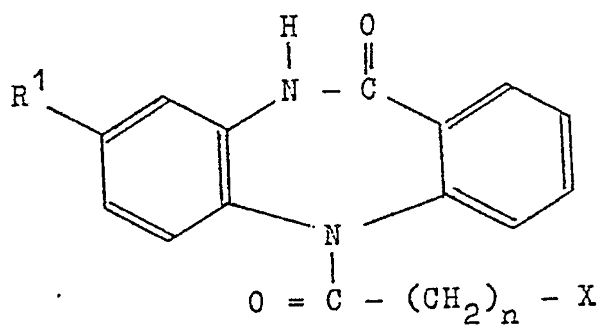

(II),

worin $R^1$ und n dasselbe wie in Anspruch 1
und
X ein Halogenatom bedeuten,
mit einem Amin der allgemeinen Formel (111)

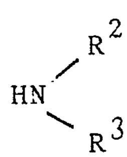

(III)

mit $R^2$ und $R^3$ wie in Anspruch 1 oder einem Salz dieses Amins oder
(b) Umsetzung eines 5,10-Dihydro-11H-dibenzo[b,e] [1,4]diazepin-11-ons der allgemeinen Formel IV

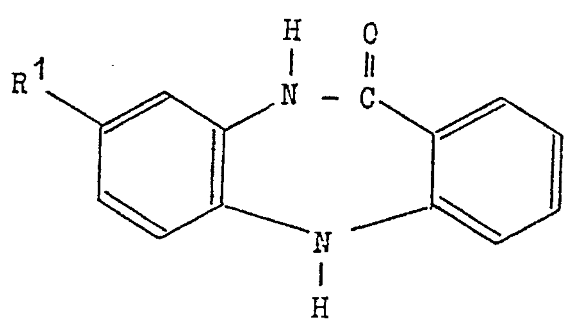

(IV)

mit $R^1$ wie oben
mit einem Aminoacylhalogenid der allgemeinen Formel V,

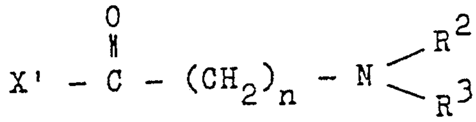 (V).

worin $R^2$, $R^3$ und n dasselbe wie oben und
X' ein Halogenatom, vorzugsweise Cl oder Br,
bedeuten, oder einem Salz dieses Aminoacylhalogenids,
(A2) zur Herstellung der Verbindungen der Formel VII
(a) Umsetzung eines 5,10-Dihydro-5-(ω-halogenacyl)-11H-dibenzo [b,e] [1,4]diazepin-11-ons der allgemeinen Formel II
wie oben definiert
mit einem Amin der allgemeinen Formel VIII

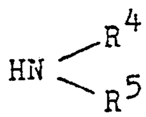 (VIII)

mit $R^4$ und $R^5$ wie in Anspruch 2
oder einem Salz dieses Amins
oder
(b) Umsetzung eines 5,10-Dihydro-11H-dibenzo b,e 1,4 diazepin-11-ons der allgemeinen Formel IV wie oben definiert mit einem Aminoacylhalogenid der allgemeinen Formel IX,

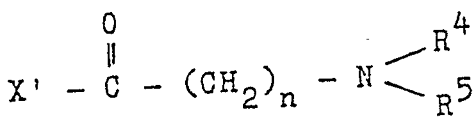 (IX),

worin $R^4$, $R^5$ und n dasselbe wie oben und
X' ein Halogenatom, vorzugsweise Cl oder Br, bedeuten,
oder einem Salz dieses Aminoacylhalogenids,
(A3) zur Herstellung der Verbindungen der Formel X
(a) Umsetzung eines 5,10-Dihydro-5-(ω-halogenacyl)-11H-dibenzo[b,e] [1,4]diazepin-11-ons der allgemeinen Formel II
wie oben definiert
mit einem Amin der allgemeinen Formel XI

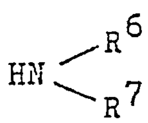 (XI)

mit $R^6$ und $R^7$ wie in Anspruch 3
oder einem Salz dieses Amins oder
(b) Umsetzung eines 5,10-Dihydro-11H-dibenzo[b,e]-[1,4]diazepin-11-ons der allgemeinen Formel IV wie oben definiert
mit einem Aminoacylhalogenid der allgemeinen Formel XII

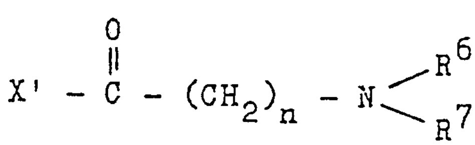 (XII)

worin $R^6$ und $R^7$ dasselbe wie oben und
$X'$ ein Halogenatom, vorzugsweise Cl oder Br,
bedeuten,
oder einem Salz dieses Aminoacylhalogenids
sowie erwünschtenfalls
(B) Überführung der erhaltenen Verbindungen der Formeln I, VII bzw. X, vorzugsweise mit physiologisch verträglichen anorganischen oder organischen Säuren, in entsprechende Säureadditionssalze
und/oder erwünschtenfalls
(C) Freisetzung der entsprechenden Basen der Formeln VII bzw. X aus Salzen dieser Verbindungen.

6. Verfahren nach Anspruch 5, gekennzeichnet durch eine oder mehrere der folgenden Maßnahmen:
- Durchführung der Umsetzungen in einem inerten organischen Lösungsmittel, das vorzugsweise ausgewählt ist unter Alkoholen wie Ethanol, n-Propanol oder Isopropanol, Ethern wie Dioxan oder Tetrahydrofuran, aromatischen Kohlenwasserstoffen wie Benzol, Toluol oder Xylol, halogenierten aliphatischen und aromatischen Kohlenwasserstoffen wie Chloroform, 1,2-Dichlorethan, Tetrachlorkohlenstoff oder Chlorbenzol oder dipolar-aprotischen Lösungsmitteln wie Dimethylformamid, Acetonitril oder Dimethylsulfoxid;
- Verwendung eines Überschusses der Verbindungen der Formeln VI, VIII bzw. XI als Lösungsmittel;
- Durchführung der Umsetzungen bei Temperaturen zwischen Raumtemperatur und 150 °C;
- Durchführung der Umsetzungen in Stufe A in Gegenwart eines Säureakzeptors, vorzugsweise Alkalicarbonaten, Alkalihydrogencarbonaten oder N,N-Dimethylanilin.

7. Pharmazeutische Mittel, insbesondere zur Behandlung von Arrhythmien, Bradyarrhythmien und cholinerg induzierten Erkrankungen, gekennzeichnet durch einen Gehalt an mindestens einer Verbindung der allgemeinen Formeln I, VII bzw. X nach den Ansprüchen 1 bis 4 oder deren Salzen mit physiologisch verträglichen anorganischen oder organischen Säuren.

8. Verfahren zur Herstellung der pharmazeutischen Mittel nach Anspruch 7, gekennzeichnet durch galenische Formulierung von Verbindungen der allgemeinen Formeln I, VII bzw. X nach den Ansprüchen 1 bis 4 oder deren Salzen mit physiologisch verträglichen anorganischen oder organischen Säuren mit inerten, nichttoxischen pharmazeutisch geeigneten Träger- oder Hilfsstoffen.

9. Verwendung der Verbindungen der allgemeinen Formeln I, VII bzw. X nach den Ansprüchen 1 bis 4 oder deren Salzen mit physiologisch verträglichen anorganischen oder organischen Säuren zur Herstellung von pharmazeutischen Mitteln zur Behandlung von Arrhythmien oder cholinerg induzierten Erkrankungen.

Europäisches Patentamt

# EUROPÄISCHER RECHERCHENBERICHT

Nummer der Anmeldung

EP 90 11 4687

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0326066 (DR. K. THOMAE GMBH)<br>* Ansprüche 1-3, 7-10 *<br>* Beispiele 10, 11 *<br>--- | 1, 2, 5, 7-9 | C07D243/38<br>A61K31/55 |
| X | EP-A-0309422 (ISTITUTO DE ANGELI S.P.A.)<br>* Seite 15, Beispiel 18 *<br>--- | 1 | |
| A,D | DE-A-3409237 (DR. K. THOMAE GMBH)<br>* Ansprüche 1, 8, 9 *<br>--- | 1, 7, 9 | |
| A,D | DE-A-3523002 (DR. K. THOMAE GMBH)<br>* Ansprüche 1, 4, 9 *<br>--- | 1, 7, 9 | |
| A | DE-A-2724478 (DR. K. THOMAE GMBH)<br>* Ansprüche 1, 8-12, 14 *<br>* Seite 21, Beispiele 14 und 16 *<br>--- | 1, 2, 5, 6 | |
| A,D | EP-A-0044989 (DR. K. THOMAE GMBH)<br>* Ansprüche 1, 8 * | 1, 5 | |
| A,D | & DE-A-3028001<br>--- | | **RECHERCHIERTE SACHGEBIETE (Int. Cl.5 )** |
| A,D | JOURNAL OF MEDICINAL CHEMISTRY, vol. 6, no. 3, Mai 1963, Seiten 255 - 261; A.M. MONRO et al.: "Some Analogs of Imipramine"<br>* Seite 259, rechte Spalte, Zeilen 43 - 61 *<br>* Seite 256, rechte Spalte, Verbindungen XIII und XVI *<br>--- | 1, 5 | C07D243/00<br>C07D471/00 |
| A,D | JOURNAL OF PHARMACOLOGY AND EXPERIMENTAL THERAPEUTICS, vol. 136, no. 1, April 1962, Baltimore, USA, Seiten 114 - 124; C.M. MOKLER et al.: "Pharmacology of a new antiarrhythmic agent, gamma-diisopropylamino-alpha-phenyl-alpha-(2-pyridyl)-butyramide (SC-7031)"<br>----- | 7 | |

Der vorliegende Recherchenbericht wurde für alle Patentansprüche erstellt

| Recherchenort | Abschlußdatum der Recherche | Prüfer |
|---|---|---|
| BERLIN | 16 NOVEMBER 1990 | HASS C. |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)